# EUROPEAN PATENT SPECIFICATION

(11) **EP 0 882 732 B1**
(45) Date of publication and mention of the grant of the patent: **03.03.2004**
(21) Application number: 97109052.7
(22) Date of filing: 04.06.1997
(51) Int. Cl.: C07H 15/04, C07H 15/26

(54) **Taxoid derivative and method of producing thereof**
Taxoid-Derivate und Verfahren zu deren Herstellung
Dérivés taxoides et procédé pour leur préparation

(43) Date of publication of application: 09.12.1998
(73) Proprietor: Ensuiko Sugar Refining Company, Limited, Kanagawa-ken, 230 (JP); Mandai, Tadakatsu, Kurashiki-shi, Okayama-ken 712 (JP)
(72) Inventor: Mandai, Tadakatsu c/o Kurashiki Uni.of Sci. & Arts, Kurashiki-shi, Okayama-ken, 712 (JP); Okumoto, Hiroshi c/o Kurashiki Univ.of Sci. & Arts, Kurashiki-shi, Okayama-ken, 712 (JP); Hara, Koji c/o Ensuiko Sugar Refining Co., Ltd, Yokohama-shi, Kanagawa-ken (JP); Mikuni, Katsuhiko c/o Ensuiko Sugar Refi. Co., Ltd, Yokohama-shi, Kanagawa-ken (JP); Hara, Kozo c/o Ensuiko Sugar Refining. Co., Ltd, Yokohama-shi, Kanagawa-ken (JP); Hamada, Hiroki c/o Okayamarika-Daigaku, Okayama-ken, 700 (JP)
(74) Representative: VOSSIUS & PARTNER

(56) References cited:
- WO-A-94/18954
- WO-A-96/11683
- N. PALMA & F. KNAUSEDER: "Pleuromutilin related metabolites produced by submerged culture of the Basidiomycetous Genus Clitopilus kummer." EUR. CONGR. BIOTECHNOL., vol. 1, 1984, pages 533-542, XP002047840
- D. B. A. DE BONT ET AL: "Synthesis and biological activity of beta-glucuronyl carbamate-based prodrugs of paclitaxel as potential candidates for ADEPT." BIOORGANIC & MEDICINAL CHEMISTRY, vol. 5, no. 2, February 1997, GREAT BRITAIN, pages 405-414, XP002047866
- K. V. RAO: "Semi-synthesis of paclitaxel from naturally occurring glycosidic percursors." JOURNAL OF HETEROCYCLIC CHEMISTRY., vol. 34, no. 2, March 1997, PROVO US, pages 675-680, XP002047867

## Description

### FIELD OF THE INVENTION

The present invention relates to a taxoid derivative and a method of producing it and, in detail, a taxoid derivative of which physiological activity and solubility in water were improved by combining glucose with paclitaxel, via a spacer, and a method of producing the said derivative.

### BACKGROUND OF THE INVENTION

Paclitaxel (trade name. Taxol)is a diterpene compound [M. C. Wani et al. : J. Am. Chem. Soc., 93. 2325 (1971)]isolated from the bark of Taxus brevifolia growing in North America and known as a powerful anticancer drug having an improved effect on uncurable cancer by a hitherto known chemical therapy. The mechanism of controling cancer with paclitaxel is unique and. while other anticancer drugs control formation of the microtubule which the main component of the spindle, that is a mitosis device, paclitaxel causes excess formation of microtubules and thereby, controls mitosis.

Although paclitaxel is a powerful anticancer drug, its solubility in water is low, and its utility as a medical drug is limited. Because of this, use of a solubilizing agent and studies and developments, etc. to enhance its solubility by preparing derivatives are being actively carried out. However, no sufficient measures to solve this matter have yet been found. For example, paclitaxel is at present administered to a patient using a solubilizing agent "Cremophore", and 1 liter of the solution is administered over 6hours every two weeks, a four-run of which is carried out, thus being a heavy burden on patients [Eric K. Rowinsky et al., CANCER RESEARCH 49, 4640 (1989)]. Moreover, side effects of the solubilizing agent have become a problem.

Further, although docetaxel (trade name: Taxotere) was developed as a paclitaxel derivative having improved solubility, the solubility of docetaxel in water is only 1.3 times that of paclitaxel [I.Ringer et al., J. Natl. Cancer Inst., 83, 288 (1991)], therefore it is not much improved.

To improve the solubility of paclitaxel, introduction of various functional groups into a side chain and the taxane ring was carried out and some compounds showed an improvement in solubility. However, the compound showing an increased physiological activity has not yet been reported.

There is no report concerning a sugar derivative of paclitaxel and only one report describing a compound comprising a xylose moiety by way of an ether linkage exists in Nature [H. Lataste et al., Proc. Natl. Acad. Sci. USA. 81, 4090 (1984)].

De Bont et al. (1997) Bioorganic & Medicinal Chemistry, 5, 405-414, describe the synthesis and biological activity of β-glucuronyl carbonate-based prodrugs of paclitaxel as potential candidates for antibody-directed enzyme prodrug therapy.

Rao KV (1997) J. Heterocyclic Chem. 34, 675-680 discloses the semi-synthesis of paclitaxel from naturally occuring glycosidic precusors.

For chemical glucosylation there are many methods, for example, as described in Chapter 3 in Series of Experimental Chemistry, 4th Edition, Volume 26. (Organic Synthesis VIII), edited by The Chemical Society of Japan. In all cases, the use ofa heavy metal salt or strong Lewis acid is necessary. However, since paclitaxel and docetaxel have an oxetane skeleton which is acid unstable and a fundamental skeleton having large steric hinderance, hitherto-known chemical glycosylation reactions do not proceed effectively. On the other hand, enzymatic glycosylation does not produce the aimed compounds because of the very low solubility in water of paclitaxel and docetaxel.

Further, 10-deacetyl-baccatin III extracted from the bark of Taxus brevifolia growing in North America. which is similar to paclitaxel, is a precursor of docetaxel. It is expected that a hydrophilic taxoid derivative may be prepared using this substance.

It is an object of the present invention to develop a sugar derivative of paclitaxel etc. showing an increase in both solubility and physiological activity and thereby, to reduce the load to patients and to provide an effective cancer treatment drug.

The present inventors have found that a paclitaxel derivative, in which glucose is combined with paclitaxel by an ether linkage via a spacer, shows increased solubility in water and physiological activity.

The present invention relates to taxoid derivatives wherein glucose is combined with paclitaxel, via a spacer and to a method for producing the said derivatives.

### DETAILED DESCRIPTION OF THE PREFERRED EMBODIMENTS

Hereinafter, practical examples of taxoid derivatives of the present invention are shown below.

Glucosyloxyacetyl-7-paclitaxel represented by the following formula (hereinafter, abbreviated to 7-S-paclitaxel),

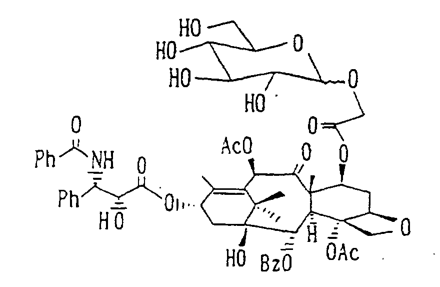

Glucosyloxyacetyl-2'-paclitaxel represented by the following formula (hereinafter, abbreviated as 2'-S-paclitaxel),

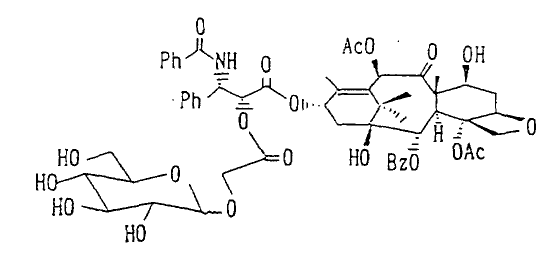

Diglucosyloxyacetyl-2',7-paclitaxe represented by the following formula (hereinafter, abbreviated to 2',7-S-paclitaxel),

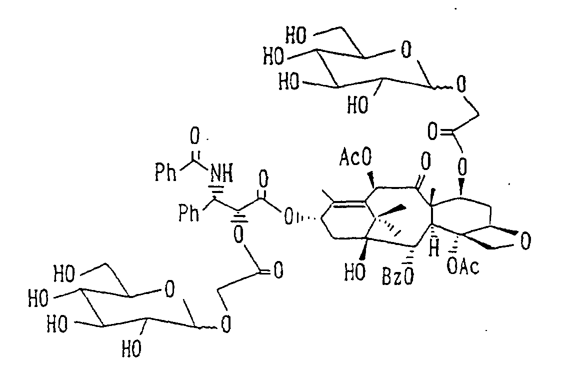

Hereinafter, the present invention is illustrated in detail. As described above, a taxoid derivative of this invention is made by combining glucose with paclitaxel, via a spacer.

Paclitaxel is obtained by isolating it from the bark of Taxus brevifolia growing in North America according to a method described in Kingston, D. G.I.: Pharmacol. Ther., 52, 1 (1992)and, in addition, paclitaxel synthesized by chemical synthesis can also be used (R.A. Holton: Europian Patent-A 400971, 1990). Also, docetaxel is derived from paclitaxel according to a method described in Greene, A. E. et al.: J.. Org. Chem. 59, 1238 (1994). 10-Deacetyl-baccatin III is a natural product extracted from Taxus brevifolia growing in North America as aforementioned.

A reaction combining glucose with paclitaxel via a spacer is carried out by using tetrabenzyl acetyloxyglucoside. This tetrabenzyl acetyloxyglucoside, an ester compound, is prepared by combining a glycolate such as ethyl glycolate etc., that is a spacer, with tetrabenzylglucose obtained by using glucose as a starting substance according to an usual procedure, followed by deethylation of the ester yielding the tetrabenzyl acetyloxyglucoside as a carboxylic acid compound which is represented by the following formula.

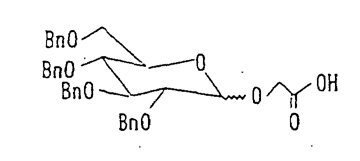

Next, one example of methods for producing tetrabenzyl acetyloxyglucoside is shown below.

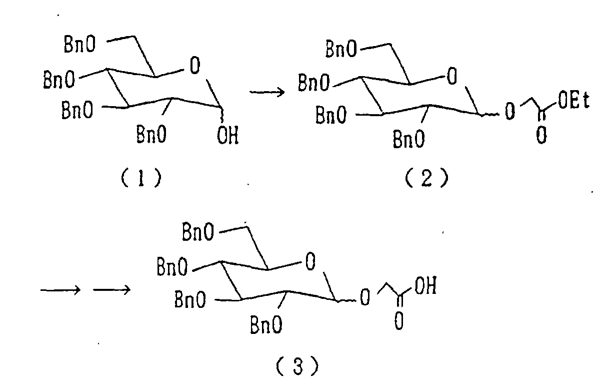

An ethyl ester (compound (2), molecular weight 626.76) is obtained by the method that tetrabenzylglucose (1) obtained according to an usual procedure is treated with ethyl glycolate and p-toluenesulfonic acid in benzene at 0-150°C, preferably 110°C, for 0.5-50 hours, preferably 8 hours, so that the 1 position of tetrabenzylglucose (1) reacts with ethyl glycolate. Then. after treating the ethyl ester (2) in an alkali (for example. 6N-NaOH) in a methanol-dioxane solution at from room temparature to 100 °C for 0.5-50 hours, preferably 3 hours, this reaction mixture is made to acidic by hydrochloric acid (for example. 1N-HCl) to cause a deethylation reaction, whereby a carboxylic acid compound is obtained which is tetrabenzyl acetyloxyglucoside (3).

In this invention, although a glycolate such as ethyl glycolate is used as a spacer of a sugar donor, by changing the alkyl chain length of this substance, the length of the spacer can be easily adjusted. For example, it is possible to use 3-hydroxybutyric acid and so forth as a spacer.

A taxoid derivative of this invention can be produced by allowing any one of the aforementioned paclitaxel to react with tetrabenzyl acetyloxyglucoside. As practical examples of methods for producing taxoid derivatives, there are methods shown in the below-described reaction process (I)

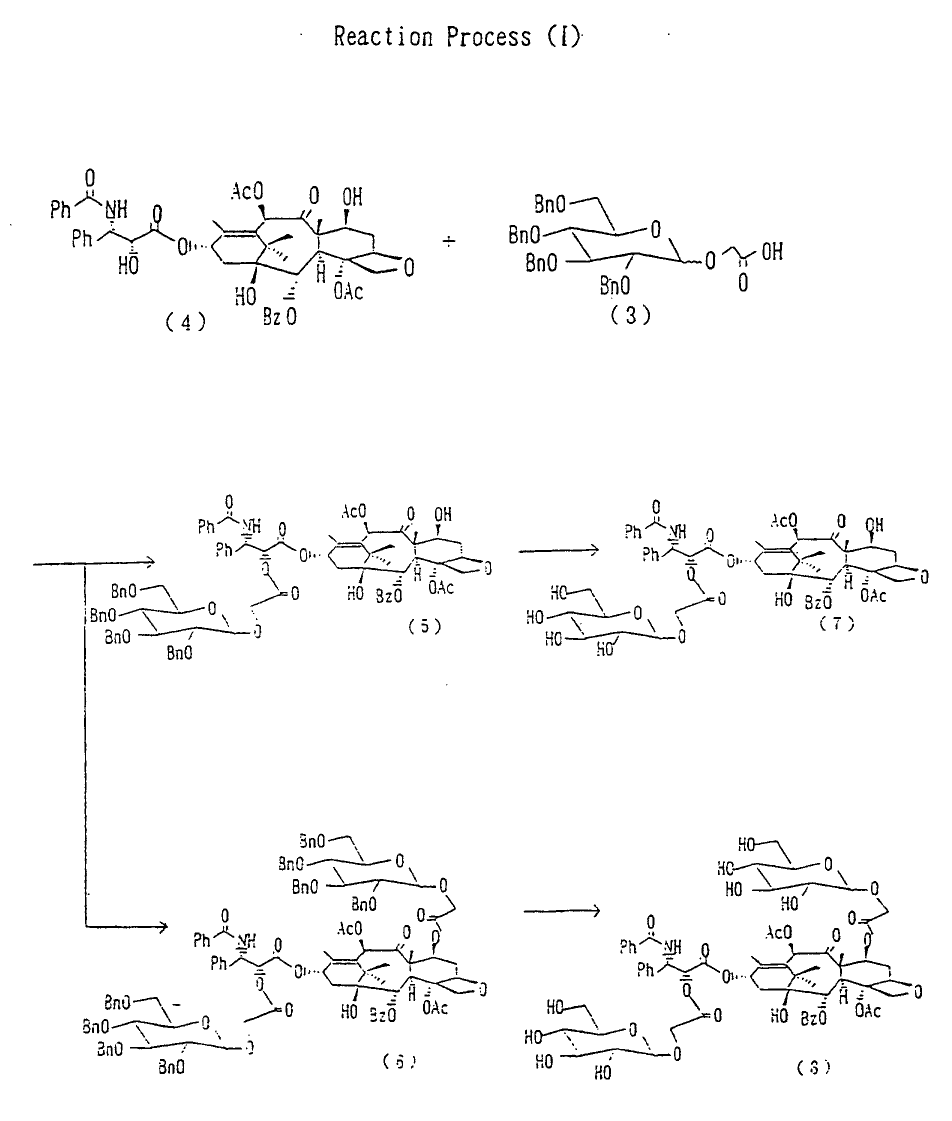

The method shown in the reaction process (I) is such that debenzylation is carried out after allowing paclitaxel (4) to react with tetrabenzyl acetyloxyglucoside (3) and, according to this method, 2'-S-paclitaxel (7) and 2',7-S-paclitaxel (8) are obtained.

That is, paclitaxel (4) and tetrabenzyl acetyloxyglucoside (3) are allowed to react with a base such as 4-dimethylaminopyridine (DMAP) etc., a condensing reagent such as dicyclohexylcarbodiimide (DCC) etc. and a solvent such as methylene chloride etc. under an argon atmosphere at room temperature for 0.5-100 hours, preferably 16.5 hours, whereby the glucoside (5) or (6) is obtained.

Next, in order to carry out the debenzylation the compound (5) or (6) is allowed to react with a catalyst such as palladium black etc. and an acid such as acetic acid etc. under a hydrogen atmosphere at room temperature with vigorous stirring for 0.5-50 hours, preferably 5 hours, whereby 2'-S-paclitaxel (7) and 2',7-S-paclitaxel (8) are obtained.

Also, the method (II) shown by the below-described reaction process is such that, after protecting the 2'-position of paclitaxel by using a chlorotriethylsilyl group, a reaction with tetrabenzyl acetyloxyglucoside followed by debenzylation and detriethylsilylation are carried out to produce a paclitaxel derivative.

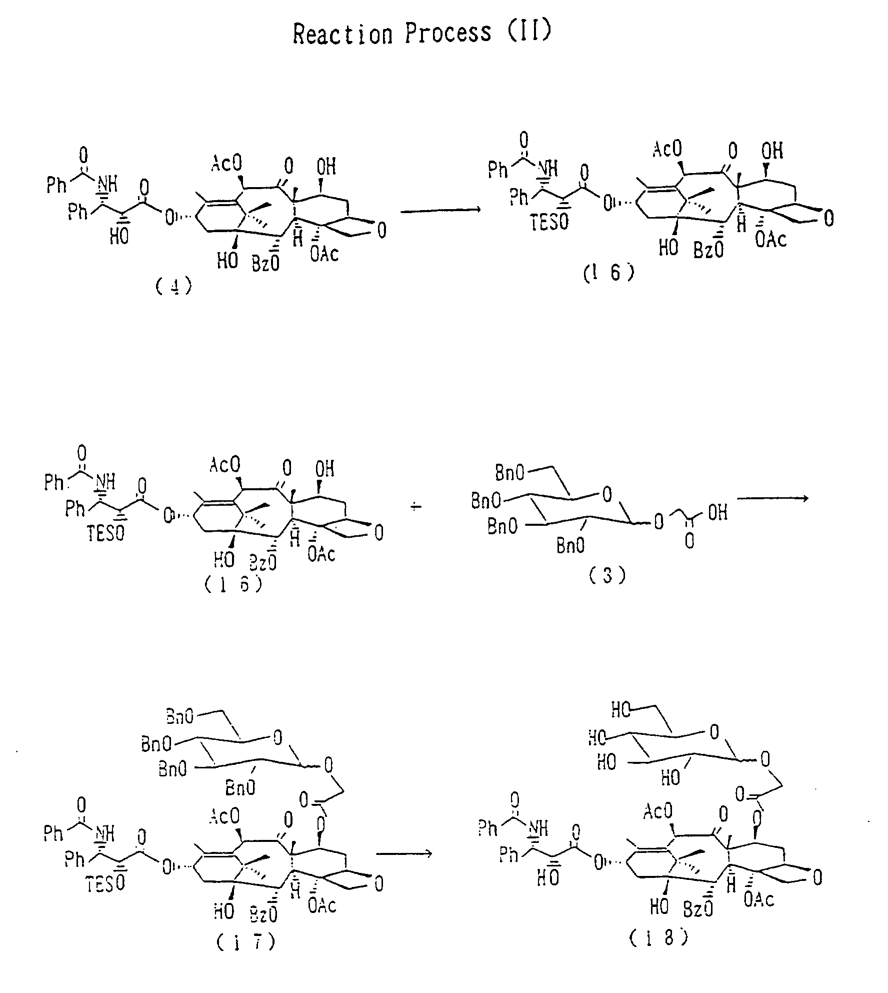

At first, paclitaxel (4) and a protecting reagent such as chlorotriethylsilane (TESCl) etc., a base such as imidazole etc. and a solvent such as dimethylformamide (DMF) etc. are allowed to react under an argon atmosphere at room temperature for 0.5-100 hours, preferably 19. 5 hours, whereby the 2' position of paclitaxel is protected by triethylsilane and the compound (16) is obtained.

Next, this obtained compound and tetrabenzyl acetyloxyglucoside (3), a base such as DMAP etc.. a condensing reagent such as DCC etc. and a solvent such as methylene chloride etc. are allowed to react under an argon atmosphere at room temperature for 0.5-100 hours, preferably 5 hours, whereby the glycoside (17) is obtained.

Next. the glycoside (17) and a catalyst such as palladium black etc. and an acid such as acetic acid etc. are allowed to react under a hydrogen atmosphere at room temperature with vigorous stirring for 0.5-50 hours. prefrably 5 hours, and to this reaction mixture a solvent such as tetrahydrofuran (THF) etc. and water are added to carry out a reaction at room temperature for 0.5-50 hours, prefrably 15 hours. whereby the aimed compound (18) is obtained which is 7-S-paclitaxel represented by the above formula.

Taxoid derivatives of this invention can be separated easely into an anomer by applying liquid chromatography using a carrier having silica gel such as ODS etc. and thus, a purified sample is obtained which can be used as a medicine.

These taxoid derivatives all show increased solubility in water and, while the solubility of paclitaxel is 0.4µg/ml, that of 7-S-paclitaxel is 14.7µg/ml (36.8 times), 2'-S-paclitaxel 30.6µg/ml (76.5 times) and 2',7-S-paclitaxel 48.4µg/ml (121 times). These paclitaxel derivatives also show increased solubility in alcohol.

Also, when the relative physiological activity of these paclitaxel derivatives is evaluated, taking the activity of inhibiting the depolymerization of the microtubule as 100, 7-S-paclitaxel is 225, 2'-S-paclitaxel 100 and 2',7-S-paclitaxel 77.7. Therefore, physiological activity of each paclitaxel derivative is sufficiently maintained and it is possible to use taxoid derivatives of this invention as an anticancer drugs. When galactose or mannose is used as the sugar because they have affinity with hepatocytes, derivatives effective for the medical treatment of liver cancer are obtained.

The present invention provides a taxoid derivative which shows a high solubility in water and improved physiological activity and a method for producing it. It is expected that the taxoid derivative reduces the burden on patients and may be used as an effect ive drug for the treatment of cancer.

### EXAMPLE

The present invention will be illustrated in more detail by means of the following examples.

### Production Example 1

A mixture of 1.62 g of tetrabenzylglucose (1) obtained by the conventional method, 1.56 g of ethyl glycolate, 0.10 g of p-toluenesulfonic acid and 80 ml of benzene was allowed to react under reflux at 110°C for 8 hours, whereby the compound (2) (C₃₈H₄₂O₈, molecular weight 626.74) was obtained.

Next, 1.88 g of this compound was allowed to react with 10 ml of 6N-NaOH, 10 ml of methanol and 15 ml of dioxane at from room temperature to 100°C for 3 hours. This mixture was transferred into 80 ml of 1N-HCl to carry out deethylation. whereby the compound (3) that is a carboxylic acid compound (C₃₆H₃₈O₈, molecular weight 598.69) was obtained.

The compound (3) was dissolved into deuteriumchloroform and analyzed by ¹H-NMR, and each peak was assigned to determine its structure and thus, structure of the compound was confirmed as the above-described.

### Example 1

A mixture of 256 mg of paclitaxel (4) (C₄₇H₅₁NO₁₄, molecular weight 853.92). 539 mg of tetrabenzyl acetyloxyglucoside (3) obtained from Production Example 1, 110 mg of 4-dimethylaminopyridine (DMAP), 186 mg of dicyclohexylcarbodiimide (DCC) and 8 ml of methylene chloride was allowed to react under an argon atmosphere at room temperature for 16.5 hours, whereby a compound having a glucoside at the 2' position (5) (C₈₃H₈₇NO₂₁, molecular weight 1434.59) and a compound having a glucoside at the 2', 7 positions (6) (C₁₁₉H₁₂₃NO₂₈, molecular weight 2015.27) were obtained.

Debenzylation of 187 mg of the compound (5) was carried out by reacting with 50 mg of palladium black and 3 ml of acetic acid under a hydrogen atmosphere at room temperature with vigorous stirring for 5 hours, whereby 101 mg of 2'-S-paclitaxel (7) (C₅₅H₆₃NO₂₁, molecular weight 1074.10) were obtained. The yield was 73 %. Also, debenzylation of 983 mg of the compound (6) was carried out by reacting with 200 mg of palladium black and 3 ml of acetic acid under a hydrogen atmosphere at room temperature with vigorous stirring for 5 hours, whereby 259 mg of 2',7-S-paclitaxel (8) (C₆₃H₇₅NO₂₈, molecular weight 1294.28) were obtained. The yield was 41 %.

Next, using a column (φ 20mm, volume 40 ml) filled by silica gel (trade name:Kieselghur, made by Merck Co., Ltd.) and chloroform as a mobile phase. 2'-S-paclitaxel and 2',7-S-paclitaxel were separately purified.

### Example 2

A mixture of 427 mg of paclitaxel (4), 0.1 mg of chlorotriethylsilane (TESC1), 102 mg of imidazole and 5 ml of DMF was allowed to react under an argon atmosphere at room temperature for 19.5 hours, whereby a compound protected by a triethylsilyl group at the 2' position of paclitaxel (16) (C₅₃H₆₅NO₁₄Si, molecular weight 968.18) was obtained. A mixture of 392 mg of this compound (16). 479 mg of tetrabenzyl acetyloxyglucoside (3), 98 mg of DMAP, 165 mg of DCC and 8 ml of methylene chloride was allowed to react under an argon atmosphere at room temperature for 5 hours, whereby the glucoside (17) (C₈₉H₁₀₁NO₂₁Si, molecular weight 1548.86) was obtained.

Next, 513 mg of the obtained compound (17) with 100 mg of palladium black and 3 ml of acetic acid were allowed to react under a hydrogen atmosphere at room temperature with vigorous stirring for 5 hours and further, 1 ml of tetrahydrofuran (THF) and 1 ml of water were added to the reaction mixture, which was then allowed to react at room temperature for 15 hours to obtain 350 mg of 7-S-paclitaxel (18) (C₅₅ H₆₃NO₂₁, molecular weight 1074. 10).

Next, using a column (φ 20mm x 250mm) filled by silica gel (trade name: ODS, made by YMC Co., Ltd.) and methanol as a mobile phase, each anomer of 7-S-paclitaxel was purified.

7-S-paclitaxel was dissolved into deuteriumchloroform and analysed by ¹H-NMR and the structure was determined by assigning respective peaks. Results are shown below.

¹H-NMR of 7-S-paclitaxel (α-anomer) (500 MHz, CDCl₃) 1.12 (s, 3H, CH₃), 1.18 (s, 3H, CH₃), 1.77 (s, 3H, CH₃), 1.83 (s. 3H, CH₃), 2.15(s, 3H, CH₃), 2.35 (s, 3H, CH₃), 1.6-2.55 (m. 5H), 3.4-3.9 (m. 7H). 4.0-4.4 (m, 4H), 4.75-5.1 (m, 3H), 5.5-5.8 (m, 3H), 6.05-6.2 (m, 1H), 7.2-7.6 (m. 11H, Ar, NH), 7.6-7.7 (m. 1H, Ar), 7.7-7.9 (m. 2H, Ar), 8.1-8.2 (m, 2H. Ar)

¹H-NMR of 7-S-paclitaxel (β-anomer) (500 MHz, CDCl₃) 1.14 (s, 3H. 17-CH₃), 1.20 (s, 3H, CH₃), 1.81 (s. 3H, CH₃), 1.84 (s, 3H, CH₃), 2.17 (s, 3H, CH₃CO), 2.38 (s, 3H. CH₃CO), 2.25-2.35 (m, 2H), 2.5-2.7 (m, 2H), 3.3-3.9 (m, 5H), 4.1-4.5 (m, 4H), 4.85 (br, 1H, H2'), 4.95 (brd. J = 9.1, 1H, H5), 5.5-5.8 (m, 3H), 6.1-6.2 (m, 2H), 7.3-7.6 (m, 11H, Ar, NH), 7.6-7.7 (m, 1H, Ar), 7.7-7.8 (m, 2H. Ar), 8.1-8.2 (m. 2H, Ar)

### Example 3

Ten milligram of paclitaxel, 7-S-paclitaxel, 2'-S-paclitaxel and 2', 7-S-paclitaxel was separately weighted and 5 ml of water were added to each compound, which was stirred for 18 hours. After stirring, the supernatant was filtered by a membrane filter (0.45µm) and a filtate was analyzed by HPLC. As a result, solubility in water of each compound was as shown in Table 1. Further, analysis conditions were as described below.
Colum: Taxil 5µ (4.6 x 250mm). made by MetaChem
Solvent: MeOH/H₂O (80/20)
Flow rate: 0.5 ml/min
Detector: photodiode detector (230 nm)
Injected amount: 20 µl

**Table 1**

| Sample | Solubility (µg/ml) |
|---|---|
| Paclitaxel | 0.4 |
| 7-S-paclitaxel | 14.7 |
| 2'-S-paclitaxel | 30.6 |
| 2',7-S-paclitaxel | 48.4 |

As clearly shown in the Table compared with paclitaxel, the solubility of paclitaxel derivatives show strikingly high values. However, it was recognized that 2'-S-paclitaxel and 2',7-S-paclitaxel are decomposed to paclitaxel in aqueous solutions and are unstable in aqueous solutions.

### Example 4

Paclitaxel, 7-S-paclitaxel, 2'-S-paclitaxel and 2',7-S-paclitaxel were separately dissolved into dimethylsulfoxide (DMSO), and an inclusion complex (made by Ensuiko Sugar Refining Co., Ltd.) of dimethyl-β-cyclodextrin (DM-β-CD, made by Ensuiko Sugar Refining Co., Ltd.) with paclitaxel was dissolved in water so that the concentrations of these compounds in the reaction solutions were adjusted at 5µM.

Next, each of the above-described samples is mixed with tubulin (a main constituting protein of the microtubule) and allowed to react at 37°C for 15 minutes. Absorbance at 350 nm of the reaction solution was measured at 2, 5, 10 and 15 minutes after initiation of the reaction. Also, after the reaction ended, calcium chloride was added and. 5 minutes after its addition, absorbance at 350 nm was again measured. From each measured value the relative activity of each sample was determined such that polymerization-promoting activity and depolymerization - inhibiting activity were taken as 100, and the results are shown in Table 2.

As clearly shown in the Table, the depolymerization-inhibiting activity of 7-S-paclitaxel is more than twice as potent as that of paclitaxel and it was confirmed that 7-S-paclitaxel is a very effective anticancer drug. Also, it was recognized that the polymerization-promoting activity is enhanced by making a complex which includes paclitaxel in DM- β-CD.

**Table 2**

| Sample | Polymerization-promoting activity | Depolymerization-inhibiting activity |
|---|---|---|
| Paclitaxel | 100 | 100 |
| 7-S-paclitaxel | 91.9 | 225 |
| 2'-S-paclitaxel | 52.7 | 100 |
| 2',7-S-paclitaxel | 40.5 | 77.7 |
| DM-β-CD-paclitaxel | 123 | 62.7 |

## Claims

1. A taxoid derivative from glucosyloxyacetyl-7-paclitaxel represented by the following formula: 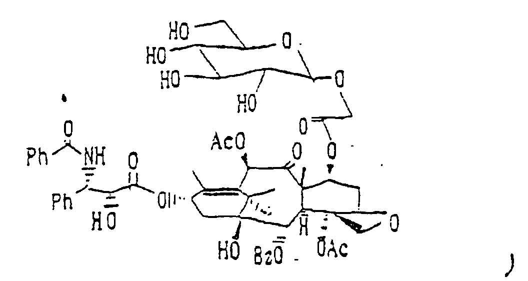 glucosyloxyacetyl -2'-paclitaxel represented by the following formula: 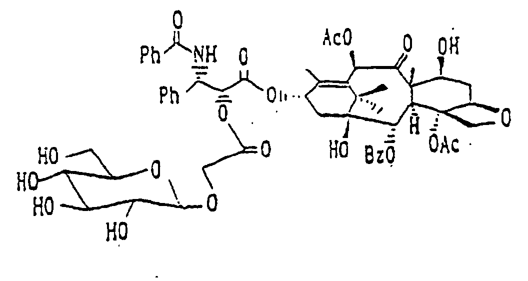 and
diglucosyloxyacetyl -2',7-paclitaxel represented by the following formula:

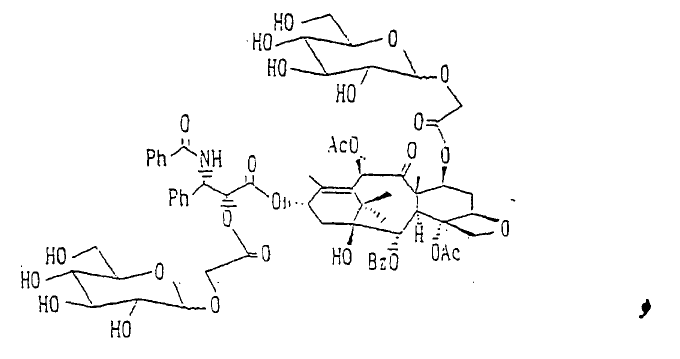

2. A method of producing a taxoid derivative of claim 1 comprising combining glucose with paclitaxel, via glycolate as a spacer using tetrabenzylacetyloxyglucoside.

3. A method according to claim 2 comprising protecting a hydroxyl group at the 2' position of a paclitaxel by chlorotriethylsilane followed by reacting said paclitaxel with tetrabenzyl acetyloxyglucoside followed by the removal of benzyl and triethylsilyl groups for producing glucosyloxyacetyl-7-paclitaxel.

4. A method according to claim 2 of reacting paclitaxel with tetrabenzyl acetyloxyglucoside followed by the removal of benzyl groups for producing glucosyloxyacetyl-2'-paclitaxel, or diglucosyloxyacetyl-2',7-paclitaxel.

## Patentansprüche

1. Taxoidderivat von Glucosyloxyacetyl-7-paclitaxel der nachstehenden Formel: 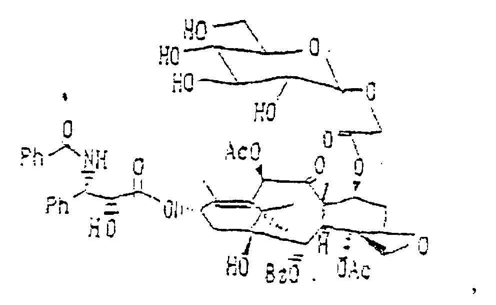 Glucosyloxyacetyl-2'-paclitaxel der nachstehenden Formel: 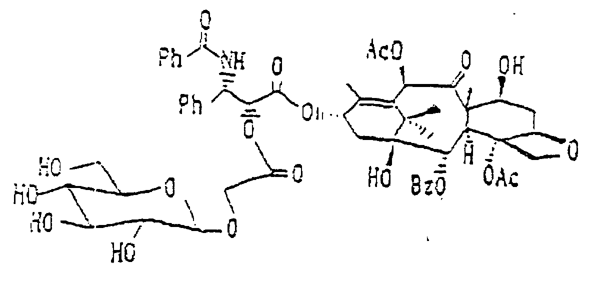 und
Diglucosyloxyacetyl-2',7-paclitaxel der nachstehenden Formel:

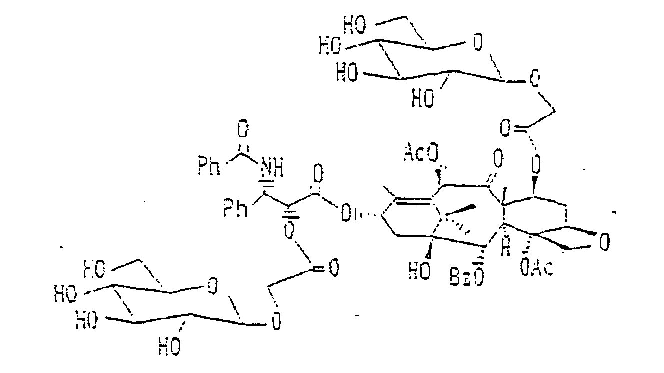

2. Verfahren zur Herstellung eines Taxoidderivats gemäß Anspruch 1, umfassend Kombinieren von Glucose mit Paclitaxel über ein Glycolat als Spacer unter Verwendung von Tetrabenzylacetyloxyglucosid.

3. Verfahren gemäß Anspruch 2, umfassend Schützen der Hydroxylgruppe an der 2'-Position von Paclitaxel durch Chlortriethylsilan gefolgt von Umsetzen des Paclitaxels mit Tetrabenzylacetyloxylglucosid gefolgt von Entfernen der Benzyl- und Triethylsilylgruppen, um Glucosyloxyacetyl-7-paclitaxel herzustellen.

4. Verfahren gemäß Anspruch 2, zum Umsetzen von Paclitaxel mit Tetrabenzylacetyloxyglucosid gefolgt von Entfernen der Benzylgruppen, um Glucosyloxyacetyl-2'-paclitaxel oder Diglucosyloxyacetyl-2',7-paclitaxel herzustellen.

## Revendications

1. Dérivé de taxoïde choisi par le glucosyloxyacétyl-7-paclitaxel représenté par la formule suivante : 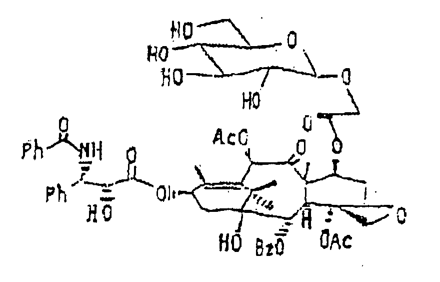 le glucosyloxyacétyl -2' -paclitaxel représenté par la formule suivante : 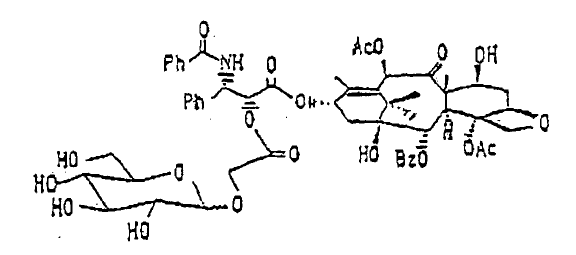 et
le diglucosyloxyacétyl-2',7-paclitaxel représenté par la formule suivante :

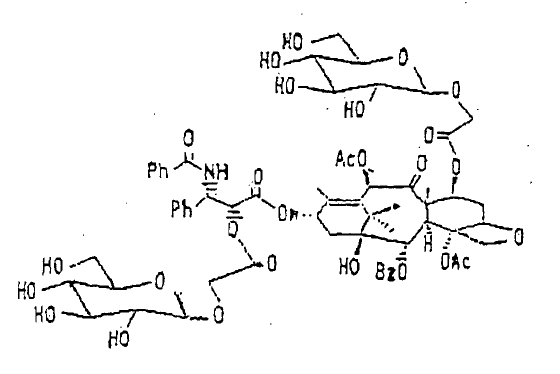

2. Méthode de préparation d'un dérivé de taxoïde selon la revendication 1, qui comprend une étape de combinaison de glucose et de paclitaxel en utilisant un glycolate à titre d'espaceur, en utilisant du tétrabenzyle acétyloxyglucoside.

3. Méthode selon la revendication 2, comprenant une étape de protection d'un groupe hydroxyle d'un paclitaxel en position 2' par du chlorotriéthylsilane, suivie par une étape consistant à faire réagir ledit paclitaxel avec du tétrabenzyle acétyloxyglucoside, suivie par une étape d'élimination des groupes benzyle et triéthylsilyl, pour produire du glucosyloxyacétyl-7-paclitaxel.

4. Méthode selon la revendication 2 de réaction du paclitaxel avec du tétrabenzyle acétyloxyglucoside suivie par une élimination des groupes benzyle pour produire du glucosyloxyacétyl-2'-paclitaxel ou du diglucosyloxyacétyl-2',7-paclitaxel.
